## Europäisches Patentamt
## European Patent Office
## Office européen des brevets

(19)

(11) Numéro de publication : **0 251 872 B1**

(12)

# FASCICULE DE BREVET EUROPEEN

(45) Date de publication du fascicule du brevet :
**09.01.91 Bulletin 91/02**

(51) Int. Cl.⁵ : **E21B 33/03, E21B 34/02, E21B 43/12, B09B 3/00**

(21) Numéro de dépôt : **87401408.7**

(22) Date de dépôt : **22.06.87**

(54) **Tête de puits à debit réglable pour exploitation de biogaz.**

(30) Priorité : **23.06.86 FR 8609032**

(43) Date de publication de la demande :
**07.01.88 Bulletin 88/01**

(45) Mention de la délivrance du brevet :
**09.01.91 Bulletin 91/02**

(84) Etats contractants désignés :
**BE CH DE ES FR GB IT LI NL**

(56) Documents cités :
**DE-A- 3 131 100
FR-A- 2 388 986
US-A- 1 767 958
US-A- 1 949 323
US-A- 4 047 695
US-A- 4 416 328**

(73) Titulaire : **BIOGAZ SYSTEM DEPOLLUTION VALORISATION SARL - B.S.D.V.
Le Moulin des Bordes Lantages
F-10210 Chaource (FR)**

(72) Inventeur : **Morizot, Denis
Le Moulin des Bordes Lantages
F-10210 Chaource (FR)**

(74) Mandataire : **Kügele, Bernhard
NOVAPAT-CABINET CHEREAU 63 bis,
boulevard Bessières
F-75017 Paris (FR)**

## Description

La présente invention a pour objet une tête de puits utilisable pour l'exploitation du biogaz produit par la décomposition des matières organiques notamment dans les décharges d'ordures.

Les décharges d'ordures plus particulièrement les décharges d'ordures ménagères contiennent pour une grosse part, des matières organiques susceptibles de dégager par un processus de dégradation biologique (fermentation, décomposition) du biogaz. Ce processus se développe de façon autonome et spontanément à l'abri de l'air dès que la quantité de déchets est suffisante. Ces gaz diffusent à travers la masse de déchets et s'échappent dans l'atmosphère. Une tonne de déchets contient environ 100 kg de matières organiques qui donnent après dégradation 160 mètres cubes de gaz dont 80 mètres cubes de méthane pur.

De façon à supprimer les nuisances par les émanations et de façon à récupérer l'énergie disponible, il a été proposé de capter et d'exploiter le biogaz in situ. Le biogaz est collecté et conduit par pompage vers un brûleur de façon à récupérer les calories ou transformer cette énergie en électricité. Le biogaz peut être collecté par circulation naturelle mais il a en effet été jugé plus rentable de forcer cette circulation par un système de pompage. Quant aux puits existants ils sont prévus pour une décharge stabilisée et ne sont pas modifiables. Dans le cas de changement de niveau, il faut extraire tout le matériel en place et réaliser une nouvelle installation sur la décharge une fois modifiée.

Les décharges actuelles évoluent rapidement compte tenu de l'augmentation des déchets et de la diminution des surfaces disponibles. Le principe d'exploitation consiste à superposer des couches d'ordures sur une épaisseur à peu près constante de quelques mètres que l'on recouvre d'une couche de terre de faible épaisseur pour limiter tant les émanations que les effets d'érosion dus au vent qui provoquent la dispersion des déchets. Lorsque le niveau de la décharge doit être rehaussée, la couche de terre est momentanément repoussée, les nouveaux déchets sont alors répandus sur toute la surface et la couche de terre est alors rapportée.

Le biogaz généré par le volume de déchets qui entoure le puits circule dans un plan horizontal en direction de la sonde de collecte située à l'intérieur du puits sous l'effet de la dépression due au pompage. Le principe même de l'exploitation de telles décharges et du biogaz qui en est issu pose le problème de la répartition car le biogaz n'est en effet pas produit dans les mêmes quantités en tous les endroits. De plus, malgré les dispositifs efficaces d'étanchéité tels que des cônes tronqués placés sur les enveloppes de puits ou la terre rapportée à la surface des déchets, il peut y avoir en cas de dépression trop forte due à un pompage trop intense des pénétrations d'air donc d'oxygène, qui se mélange aux composants essentiels du biogaz à savoir méthane et gaz carbonique. Sur un même puits ce taux de mélange air-biogaz pourra varier simplement par la qualité de la terre rapportée ou bien par la compacité des déchets qui viennent d'être répandus.

Les têtes de puits doivent donc pouvoir s'adapter in situ aux conditions d'exploitation. Cette nécessité est d'autant plus vraie dans le cas des puits progressifs dont les paramètres varient régulièrement.

Les différents puits disposés sur une décharge sont reliés entre eux en série par un réseau de canalisations. L'extrémité du réseau est elle-même raccordée au système d'exploitation proprement dit : pompes, torchères, brûleurs, appareils de contrôle et éventuellement turbines, généralement situés à une distance de plusieurs centaines de mètres. Malgré des dispositifs sophistiqués de supportage des canalisations, les dilatations peuvent provoquer des détériorations des têtes de puits lorsque la canalisation du réseau est raccordée directement et de façon rigide à la paroi de la tête de puits.

Par ailleurs, lors de l'exploitation l'eau contenue dans le biogaz se condense au niveau de la tête de puits ainsi que dans les canalisations du réseau. Cette eau provoque alors une réduction de diamètre et donc de débit dans les canalisations en point bas et elle s'accumule simultanément dans les chambres de la tête de puits.

C'est le but de l'invention de proposer une tête de puits à débit réglable (voir US-A-4416328) plus particulièrement caractérisée en ce qu'elle comporte deux chambres l'une supérieure en liaison directe avec l'enveloppe de puits, l'autre inférieure avec le réseau. Ces deux chambres pouvant communiquer à travers un dispositif réglable à pointeau.

Ce dispositif à pointeau est accessible de l'extérieur pour permettre le réglage une fois la tête de puits montée in situ.

Un piquage permet l'introduction d'appareillages de contrôle et de réglage tels que anémomètre, oxymètre, dépressiomètre ou thermomètre.

La tête de puits comporte d'autre part une portion de canalisation qui traverse la chambre inférieure, canalisation aux extrémités de laquelle sont disposées des brides de fixation qui permettent de la relier au réseau. Des alésages sont usinés dans la portion de canalisation interne à la chambre afin de faire communiquer cette dernière avec le réseau. Ces alésages sont disposés le long de la génératrice inférieure de la canalisation, de façon que l'eau de condensation puisse également s'écouler par ces orifices.

La tête de puits selon l'invention comporte un dispositif de trop-plein pour l'évacuation en continu de l'eau de condensation qui provient des canalisations et de la tête de puits elle-même sans que la dépression soit modifiée.

La présente invention sera mieux comprise à la lecture de la description qui va suivre d'un mode de réalisation particulier qui fera apparaître d'autres avantages, en accord avec les figures annexées qui représentent

Figure 1 : coupe selon un plan vertical d'une tête de puits selon l'invention ;

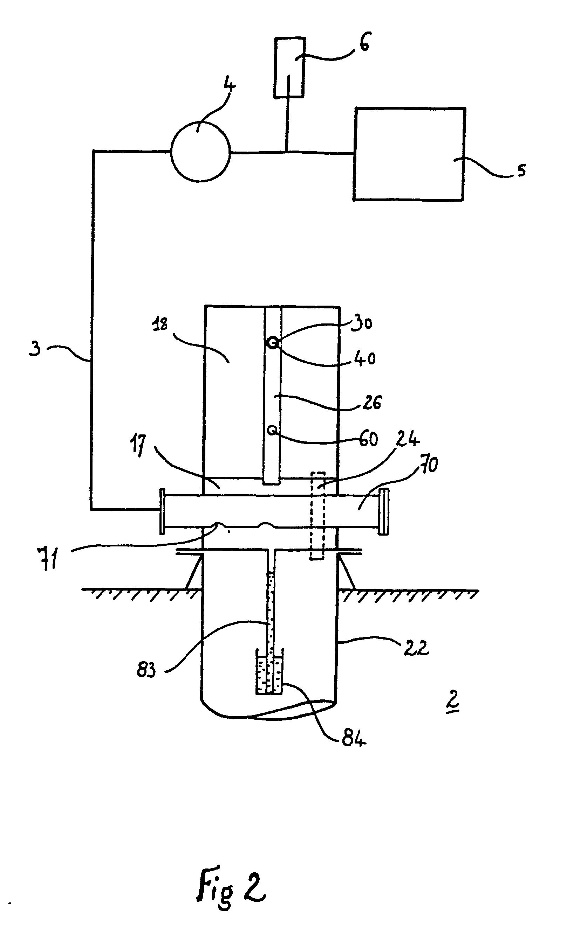

Figure 2 : schéma d'une installation en exploitation comportant des têtes de puits selon l'invention.

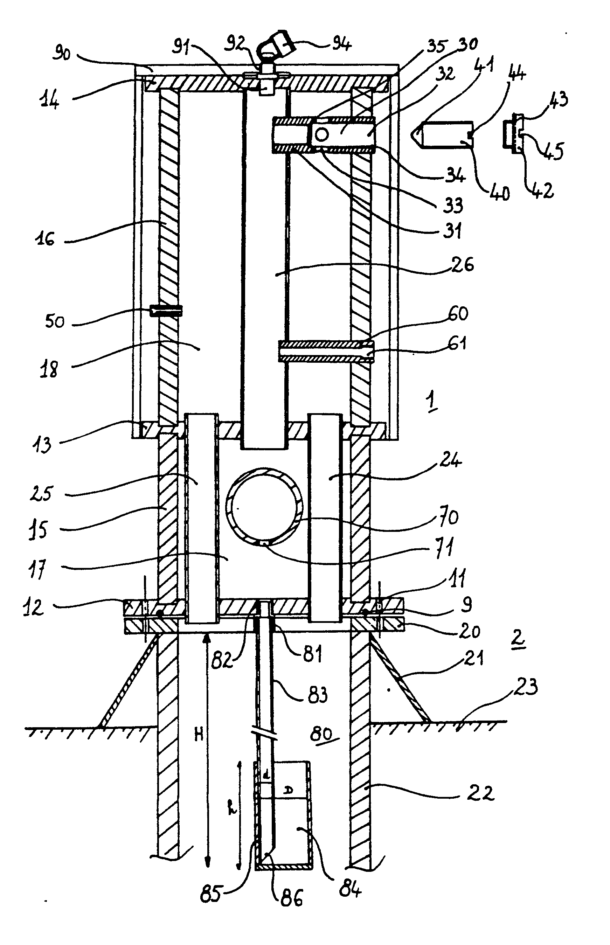

Sur la figure 1 la tête de puits 1 est montée sur une enveloppe de puits progressif 2. La bride 20 et la plaque 12 sont assemblées par vissage 11 de façon à les rendre solidaires, l'étanchéité étant assurée par un joint circulaire 9. L'enveloppe de puits 2 est munie d'un cône tronqué 21 associé à la partie cylindrique 22 de l'enveloppe, la base de ce cône reposant sur la surface 23 de façon étanche.

La tête de puits 1 est constituée de trois plaques 12, 13, 14 et deux viroles associées 15 et 16 de façon à définir deux chambres 17, 18 superposées et séparées par une plaque unique et commune 13. La chambre 17 située immédiatement au-dessus du puits sera appelée chambre inférieure et la chambre superposée 18 sera appelée chambre supérieure.

Deux tubes 24 et 25 traversent la chambre inférieure ainsi que les deux plaques 12 et 13 qui la constituent, de façon étanche mettant ainsi en communication la chambre supérieure avec l'intérieur de l'enveloppe de puits.

Un tube central 26 soudé à sa partie supérieure à la plaque 14 traverse la chambre supérieure pour déboucher dans la chambre inférieure à travers la plaque 13. Entre la virole 16 de la chambre supérieure et le tube central 26 est disposé un piquage 30 de façon que l'une des extrémités 31 du piquage débouche dans le tube central et que l'autre extrémité 32 débouche à l'extérieur de la chambre supérieure. Ce piquage de forme cylindrique est percé d'alésages 33 dans sa partie comprise dans l'espace annulaire entre le tube central et la virole. Ces alésages sont régulièrement répartis selon un cercle. La partie du piquage dirigé vers l'extérieur est filetée en 34 pour recevoir le corps d'un pointeau 40 dont la partie conique 41 coopère avec une portée chanfreinée 35 usinée dans le corps du piquage. Un bouchon à vis 42 obture ce piquage de façon étanche par l'intermédiaire du joint 43. Les fentes 44 et 45 permettent à l'aide d'un même outil de régler le pointeau ou de serrer le bouchon.

Un piquage 50 permet d'introduire un dépressiomètre afin de vérifier la dépression dans la chambre supérieure.

Un bouchon 51 et joint 52 assurent l'étanchéité.

Un piquage 60 avec embout fileté standard 61, bouchon 62 et joint 63 met en relation l'intérieur du tube central avec l'extérieur de la tête de puits. Il est possible de connecter à un tel piquage un oxymètre, un dépressiomètre, un anémomètre ou bien une sonde pour prélèvements d'échantillons en vue d'analyses par chromatographie ou tout autre appareil nécessaire lors du réglage du dispositif comme cela sera décrit ultérieurement.

La virole 15 est traversée selon un diamètre par une portion de canalisation 70 dont la longueur est supérieure au diamètre de la chambre inférieure, les extrémités de cette canalisation étant accessibles à l'extérieur de la tête de puits. Les raccords à bride non figurés permettent de relier cette canalisation au réseau. Des alésages 71 sont percés le long d'une génératrice de la canalisation plus particulièrement le long de la génératrice inférieure et ce dans la portion de canalisation située à l'intérieur de ladite chambre.

Le dispositif de trop-plein 80 est constitué d'un raccord 81 qui visse d'une part dans un alésage taraudé 82 usiné dans la plaque 12 et d'autre part dans un tube 83 dont l'extrémité supérieure est filetée. La longueur H de ce tube place son extrémité inférieure dans la partie cylindrique 22 de l'enveloppe de puits. Un pot 84 est lié au tube par une génératrice commune en 85 sur une hauteur h de façon que le tube plonge dans le pot. Le diamètre D de ce dernier est supérieur au diamètre d du tube. La partie débouchante 86 du tube est biseauté.

La tête de puits est complétée par un capot 90 de sécurité, de la hauteur de la chambre supérieure et du diamètre des deux plaques 13 et 14, qui vient protéger l'accès aux organes de réglage et aux différents piquages auxiliaires. Un pion 91 solidaire de la plaque 14 traverse le capot par un alésage ajusté 92. La partie débouchante 93 du pion est percée d'un alésage destiné à recevoir un cadenas 94 ou une barrette qui empêche le retrait du capot.

Le matériau utilisé pour la réalisation de cette tête de puits est du polyéthylène haute densité, mais tout matériau insensible à la corrosion notamment en atmosphère humide pourrait convenir par exemple un matériau composite à base de fibres et de résines ou certains alliages légers notamment d'aluminium. Le polyéthylène haute densité allie les avantages de la résistance à la corrosion d'une bonne tenue mécanique et de sa facilité de soudage et de collage.

Nous allons maintenant décrire le fonctionnement d'une installation utilisant une tête de puits selon l'invention. Une installation comprend donc au moins une tête de puits 1, une enveloppe de puits 2, un réseau de canalisations schématisé en 3 ainsi qu'une pompe 4 et une chaudière 5. L'ensemble peut être complété par une torchère 6. La pompe 4 provoque une dépression compte-tenu des différentes pertes de charge dans le réseau de 40 millibars environ. La portion de canalisation 70 se trouve donc soumise à une dépression de 40 millibars tout comme la chambre inférieure 17 par l'intermédiaire des trous 71. La colonne centrale 26 en relation directe avec la chambre inférieure est également soumise à une dépression d'environ 40 millibars . Le piquage 30 par l'intermédiaire de son pointeau 40 sera réglé de façon que la dépression dans la chambre supérieure 18 se situe dans la plage de 2 à 20 millibars . Cette dépression sera égale à la dépression qui règne dans l'enveloppe du puits puisque la chambre supérieure 17 est en liaison avec l'intérieur de l'enveloppe de puits 22 par l'intermédiaire des tubes 24. Par le piquage 60 on introduit un oxymètre qui permettra de déterminer la teneur en oxygène du gaz qui circule dans le tube central en provenance du puits. Dans le cas d'une détection de la présence d'oxygène dans le mélange, la dépression dans l'enveloppe de puits par rapport à la dépression dans le réseau sera diminuée par vissage de la vis pointeau jusqu'à ce que le taux d'oxygène dans le mélange soit réduit à un niveau acceptable.

Par ailleurs l'eau de condensation s'accumule à la partie inférieure de la chambre intérieure 17 et s'écoule dans le pot 84 par l'intermédiaire du tube 83. Afin de réaliser une garde hydraulique, la hauteur d'eau dans le tube se maintiendra constamment à un niveau tel que la colonne d'eau dans ce tube 83 compense la dépression dans la chambre inférieure.Le trop-plein d'eau s'évacuera par débordement du pot 84. En cas d'arrêt du système, la colonne d'eau diminue et le pot 84 reste rempli. Lors de la remise en route du système, la colonne d'eau va remonter dans le tube 83 en utilisant l'eau contenue dans le pot 84. On comprend donc que le diamètre D de ce pot soit supérieur au diamètre d du tube de façon à pouvoir l'alimenter sur toute la hauteur nécessaire.

**Revendications**

1. Tête de puits (1) à débit réglable plus particulièrement pour une installation de récupération du biogaz (3, 4, 5, 6), caractérisée en ce qu'elle comporte deux chambres l'une supérieure (18) en liaison directe avec l'enveloppe de puits (22), l'autre inférieure (17) en relation directe avec le réseau (3), ces deux chambres pouvant communiquer par l'intermédiaire d'un dispositif à débit réglable (30).

2. Tête de puits selon la revendication 1, caractérisée en ce que le dispositif réglable (30) est une vis pointeau (40).

3. Tête de puits selon la revendication 2, caractérisée en ce que ce dispositif réglable à vis pointeau est accessible de l'extérieur après retrait du bouchon d'étanchéité (42).

4. Tête de puits selon la revendication 1, caractérisée en ce qu'elle comporte une portion de canalisation (70) qui traverse de façon étanche la paroi de la chambre inférieure (15).

5. Tête de puits selon la revendication 4, caractérisée en ce que cette portion de canalisation est munie d'alésages (71) disposés sur sa génératrice inférieure.

6. Tête de puits selon l'une quelconque des revendications précédentes, caractérisée en ce que la chambre inférieure est munie d'un dispositif de garde hydraulique (80).

7. Tête de puits selon la revendication 6, caractérisée en ce que cette garde hydraulique est constituée d'un pot (84) solidaire de l'extrémité inférieure d'un tube (83) lui-même débouchant par un alésage (82) dans la partie inférieure de la chambre inférieure (17).

8. Tête de puits selon la revendication 7, caractérisée en ce que le diamètre D du pot (84) est supérieur au diamètre d du tube (83) afin que le volume du pot puisse correspondre au volume du tube.

9. Tête de puits selon la revendication 1, caractérisée en ce qu'elle est munie d'un piquage permettant l'introduction de différents appareillages tels que oxymètre, dépressiomètre, sonde de prélèvement pour analyse par chromatographie, dépressiomètre.

10. Tête de puits selon l'une quelconque des revendications précédentes, caractérisée en ce que le matériau utilisé pour sa réalisation est du polyéthylène haute densité.

**Ansprüche**

1. Bohrlochkopf (1) mit regelbarem Durchfluß insbesondere für eine Anlage zur Rückgewinnung von Biogas (3, 4, 5, 6) **gekennzeichnet durch** zwei Kammern, nämlich einer oberen Kammer (18) in direkter Verbindung mit einem Bohrlochgehäuse (22), und einer unteren Kammer (17) in direkter Verbindung mit dem Kanalnetz (3), wobei die beiden Kammern mittels einer Vorrichtung (30) mit regelbarem Druchfluß verbunden sind.

2. Bohrlochkopf nach Anspruch 1, **dadurch gekennzeichnet,** daß die regelbare Vorrichtung (30) eine Stellschraube (40) ist.

3. Bohrlochkopf nach Anspruch 2, **dadurch gekennzeichnet,** daß die mittels Stellschraube regelbare Vorrichtung nach Abnahme eines Dichtungsstopfens (42) von außen zugänglich ist.

4. Bohrlochkopf nach Anspruch 1, **gekennzeichnet durch** einen Kanalabschnitt (70), der die Wand (15) der unteren Kammer abgedichtet durchquert.

5. Bohrlochkopf nach Anspruch 4, **dadurch gekennzeichnet,** daß dieser Kanalabschnitt mit Bohrungen (71) versehen ist, die auf seiner unteren Erzeugenden angeordnet sind.

6. Bohrlochkopf nach einem der vorhergehenden Ansprüche, **dadurch gekennzeichnet,** daß die untere Kammer mit einer hydraulischen Schutzvorrichtung (80) versehen ist.

7. Bohrlochkopf nach Anspruch 6, **dadurch**

gekennzeichnet, daß diese hydraulische Schutzvorrichtung aus einem Behälter (84) besteht, der mit dem unteren Ende eines Rohrs (83) verbunden ist, das über eine Bohrung (82) in den unteren Teil der unteren Kammer (17) mündet.

8. Bohrlochkopf nach Anspruch 7, **dadurch gekennzeichnet,** daß der Durchmesser D des Behälters (84) größer als der Druchmesser d des Rohrs (83) ist, damit das Volumen des Behälters dem des Rohrs entsprechen kann.

9. Bohrlochkopf nach Anspruch 1, **gekennzeichnet durch** einen Einsatz, der das Einführen verschiedener Apparaturen wie eines Sauerstoffmeßgeräts, eines Unterdruckmeßgeräts oder einer Abnahmesonde zur chromatographischen Analyse ermöglicht.

10. Bohrlochkopf nach einem der vorhergehenden Ansprüche, **dadurch gekennzeichnet,** daß das Herstellungsmaterial Polyethylen hoher Dichte ist.

## Claims

1. A well head (1) having an adjustable flow rate, particularly for a biogas recovery plant (3, 4, 5, 6,), characterized in that it includes two chambers, an upper one (18) in direct connection with the well casing (22), a lower one (17) in direct connection with the piping system (3), with both chambers being in fluid communication through an adjustable flow rate device (30).

2. The well head of claim 1, characterized in that said adjustable device (30) includes a check screw (40).

3. The well head of claim 2, characterized in that said adjustable check-screw device is outwardly accessible after the sealing plug (42) has been removed.

4. The well head of claim 1, characterized in that it includes a pipe portion (70) which sealingly passes through the lower chamber (15) wall.

5. The well head of claim 4, characterized in that said pipe portion is provided with bores (71) on the lower generating line thereof.

6. The well head of any preceding claim, characterized in that said lower chamber is provided with an hydraulic guard device (80).

7. The well head of claim 6, characterized in that said hydraulic guard includes a pot (84) integral with the lower end of a tube (83) opening into the lower portion of said lower chamber (17) by means of a bore (82).

8. The well head of claim 7, characterized in that the pot (84) diameter (D) is larger than the tube (83) diameter (d) in order that the pot volume may correspond to the tube one .

9. The well head of claim 1, characterized in that it is provided with a tap allowing different equipments such as oxymeter, depressionmeter, sampling probe for chromatography analysis, to be introduced.

10. The well head of any preceding claim, characterized in that the material used for the making thereof comprises high density polyethylene.

Fig 2